# EUROPÄISCHE PATENTANMELDUNG

(11) **EP 1 591 059 A1**
(43) Veröffentlichungstag der Anmeldung: **02.11.2005**
(21) Anmeldenummer: 04405265.2
(22) Anmeldetag: 29.04.2004
(51) Int. Cl.: A61B 3/16, A61B 19/02

(54) **Applikation eines Schutzüberzugs auf ein ophthalmologische Geräteteil**

(71) Anmelder: SMT Swiss Microtechnology AG, 2562 Port (CH)
(72) Erfinder: Kanngiesser, Hartmut, 8038 Zürich (CH)
(74) Vertreter: Vogel, Dany

(57) **Zusammenfassung**

Es werden ein Applikationssystem und ein Applikationsverfahren zum Anbringen eines elastischen Schutzüberzugs (1) auf die kegelstumpfförmige Spitze (21) eines ophthalmologischen Geräteteils (2) vorgeschlagen. Der Schutzüberzug (1) ist in der Vertiefung (31) eines Applikatorteils (3) oberhalb eines Stempels (34) aus komprimierendem Material angeordnet. Der Stempel (34) ist am Boden der Vertiefung (31) angebracht und gegen die Öffnung (32) der Vertiefung (31) ausgerichtet. Die Spitze (21) ist durch die Öffnung (32) in den Schutzüberzug (1) einführbar. Beim Einführvorgang kommt eine Membran (14) des Schutzüberzugs (1) mit der Kontaktfläche (22) in Kontakt und wird an den Stempel (34) angedrückt. Die Membran (14) wird zwischen der Kontaktfläche (22) und dem Stempel (34) gepresst und Luft, die sich zwischen der Membran (14) und der Kontaktfläche (22) befindet, wird verdrängt. Zudem wird ein Haftbereich am Kragen (11) des Schutzüberzugs (1) an die Spitze (21) angedrückt und bewirkt eine Haftung des Schutzüberzugs (1) an der Spitze (21).

## Beschreibung

### Technisches Gebiet

Die vorliegende Erfindung betrifft die Applikation eines Schutzüberzugs auf ein ophthalmologisches Geräteteil. Die Erfindung betrifft insbesondere ein Applikationssystem, das einen elastischen Schutzüberzug für ein Geräteteil mit einer sich zu einer auf ein Auge aufsetzbaren Kontaktfläche konisch verjüngenden Spitze und ein Applikatorteil zum Anbringen des Schutzüberzugs auf die Spitze umfasst.

### Stand der Technik

In der Ophthalmologie sind Geräteteile wie Messköpfe, beispielsweise Tonometer, oder optische Applikatoren, wie Pachymeter, oft mit einer sich konisch verjüngenden, kegelstumpfförmigen Spitze versehen, die zum Aufsetzen auf ein Auge am verjüngten Ende eine Kontaktfläche aufweist. Die kegelstumpfförmige Spitze dieser Geräteteile ermöglicht dem behandelnden Arzt oder Optiker einerseits eine bessere Einsicht des zu behandelnden oder zu vermessenden Auges, andererseits erleichtert sie das Anbringen von Schutzüberzügen. Bei der Berührung des Auges durch die Kontaktfläche der Geräteteile kann es zur Übertragung von Tränenflüssigkeit und damit auch von Krankheitserregern von einem Patienten zu einem anderen kommen. Zudem können Keime oder Verunreinigungen, die an der Kontaktfläche haften, bei der Behandlung oder Messung ins Auge gebracht werden. Um dies zu vermeiden, werden die Geräteteile entsprechend gereinigt oder mit einem sauberen möglichst sterilen Schutzüberzug versehen. Messungen am Auge, insbesondere Augendruckmessungen, werden sehr häufig durchgeführt. Zum Beispiel werden anlässlich von Augenuntersuchungen bei fast jedem Patient Augendruckmessungen vorgenommen, wobei eine Messung im Allgemeinen bloss wenige Sekunden dauert. Die Vorbereitung der Geräteteile, insbesondere deren Spitzen, für die Messung sollte folglich auch möglichst schnell zu bewerkstelligen sein. Ansonsten muss der behandelnde Arzt oder Optiker mehrere Geräteteile zur Verfügung haben, die er wechselweise verwendet.

### Darstellung der Erfindung

Es ist eine Aufgabe der vorliegenden Erfindung, ein neues Applikationssystem und ein neues Verfahren zum Anbringen eines elastischen Schutzüberzugs auf ein Geräteteil, das eine sich zu einer auf ein Auge aufsetzbaren Kontaktfläche konisch verjüngende Spitze aufweist, vorzuschlagen. Es ist insbesondere eine Aufgabe der vorliegenden Erfindung, ein Applikationssystem und ein Applikationsverfahren vorzuschlagen, die ein schnelles, zuverlässiges und reproduzierbares Anbringen eines elastischen Schutzüberzugs auf eine sich konisch verjüngende, im wesentlichen kegelstumpfförmige Spitze mit einer Kontaktfläche zum Aufsetzen auf ein Auge ermöglichen.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Das Applikationssystem umfasst einen elastischen Schutzüberzug für ein Geräteteil, das eine sich zu einer auf ein Auge aufsetzbaren Kontaktfläche konisch verjüngende Spitze aufweist, und ein Applikatorteil zum Anbringen des Schutzüberzugs auf die Spitze.

Die oben genannten Ziele werden durch die vorliegende Erfindung insbesondere dadurch erreicht, dass das Applikatorteil eine Vertiefung mit einer Öffnung und einem Bodenteil aufweist, dass am Bodenteil ein Stempel aus komprimierendem Material angebracht ist, der vom Bodenteil in Richtung der Öffnung ausgerichtet ist, dass der Schutzüberzug oberhalb des Stempels in der Vertiefung so entfernbar angeordnet ist, dass die Spitze zum Anbringen des Schutzüberzugs durch die Öffnung in den Schutzüberzug einführbar ist, wobei ein beim Einführvorgang mit der Kontaktfläche in Kontakt kommender Kontaktbereich des Schutzüberzugs zwischen der Kontaktfläche und dem Stempel gepresst wird und Luft, die sich zwischen dem Kontaktbereich des Schutzüberzugs und der Kontaktfläche befindet, aus dem Kontaktbereich verdrängt wird. Das Verdrängen von Luft aus dem Bereich zwischen der Kontaktfläche und dem mit der Kontaktfläche in Kontakt kommenden Kontaktbereich des Schutzüberzugs hat den Vorteil, dass Messungen am Auge, beispielsweise die Messung des Augendrucks, nicht durch Lufteinschlüsse verfälscht werden. Bei Anwendungen mit optischen Applikatoren hat die Verdrängung der Luft den Vorteil, dass Lichtstrahlen nicht durch Lufteinschlüsse gebrochen oder gestreut werden.

Vorzugsweise weist der Stempel einen konvexen Andruckbereich auf, so dass der beim Einführvorgang der Kontaktfläche in Kontakt kommende Kontaktbereich des Schutzüberzugs mit zunehmender Komprimierung des Stempels in einem sich von einem ersten Kontaktpunkt aus vergrössernden Pressbereich zwischen der Kontaktfläche und dem Stempel gepresst wird. Dadurch werden Lufteinschlüsse zwischen der Kontaktfläche und dem mit der Kontaktfläche in Kontakt kommenden Kontaktbereich des Schutzüberzugs vom anfänglichen Kontaktpunkt ausgehend, im sich zunehmend vergrössernden Pressbereich verdrängt, so dass einerseits keine Inseln von Lufteinschlüssen verbleiben und andererseits keine Beschädigungen des Schutzüberzugs durch unter Druck stehende Lufteinschlüsse verursacht werden.

Vorzugsweise weist der Schutzüberzug einen Kragen auf, mit dem der Schutzüberzug an einem die Öffnung umlaufenden Randbereich des Applikatorteils über dem Stempel gehalten wird. Der Schutzüberzug weist einen Mantel auf, welcher einen sich vom Kragen zum Kontaktbereich des Schutzüberzugs konisch verjüngenden Innenraum bildet. Bei planem Kontaktbereich des Schutzüberzugs bilden der Mantel und der Kontaktbereich einen im wesentlichen kegelstumpfförmigen Innenraum. Die Formgestaltung des Innenraums des Schutzüberzugs ermöglicht es die Spitze passend in den Innenraum des Schutzüberzugs einzuführen. Dadurch, dass der Schutzüberzug mittels seines Kragens über dem Stempel gehalten wird, kann verhindert werden, dass der Kontaktbereich des Schutzüberzugs, beispielsweise in einer ungeeigneten Position, am Stempel haftet. Wenn die Spitze mit angebrachtem Schutzüberzug auf ein Auge aufgesetzt wird, dient der Kragen überdies dazu Flüssigkeiten, beispielsweise Augentropfen oder Tränenflüssigkeit, die vom Auge entlang der Spitze rinnen, aufzufangen und dadurch beispielsweise zu verhindern dass ein mit der Spitze verbundener Griff glitschig wird.

Vorzugsweise ist der mit der Kontaktfläche in Kontakt kommende Kontaktbereich des Schutzüberzugs als Membran ausgestaltet. Der Kragen weist einen dem Innenraum zugewandten Haftbereich mit glatter Oberfläche auf, die glatter ist als die Oberfläche der dem Innenraum zugewandten Seite des Mantels. Die Ausgestaltung des mit der Kontaktfläche in Kontakt kommenden Kontaktbereichs des Schutzüberzugs als dünne Membran hat den Vorteil, dass der Kontaktbereich des Schutzüberzugs sich sehr gut an Form und Oberfläche der Kontaktfläche anpasst und Messungen am Auge, insbesondere Augendruckmessungen, nur in einem vernachlässigbaren Ausmass beeinflusst. Die glatte Oberfläche des elastischen Schutzüberzugs im Haftbereich weist eine haftende, klebrige Wirkung auf. Beim Einführen wird die Spitze an den Haftbereich angedrückt, der Schutzüberzug bleibt an der eingeführten Spitze haften und kann zusammen mit dem anhaftenden Schutzüberzug durch die Öffnung des Applikatorteils aus der Vertiefung des Applikatorteils herausgezogen werden.

In einer Ausführungsvariante weist der Randbereich eine die Öffnung umlaufende Nut auf, die durch eine Grenzwand des Applikatorteils von der Öffnung abgegrenzt ist. Der Kragen umfasst ein in der Nut verlaufendes äusseres Kragenteil. Das äussere Kragenteil weist einen der Öffnung zugewandten äusseren Krageninnenbereich mit glatter Oberfläche auf, der die Grenzwand kontaktiert. Durch das in der Nut verlaufende äussere Kragenteil wird der Schutzüberzug in einer definierten Position, beispielsweise zentrisch, in der Vertiefung gehalten. Die glatte Oberfläche des äusseren Krageninnenbereichs weist eine haftende, klebrige Wirkung auf und bewirkt, dass der Schutzüberzug mit dem äusseren Krageninnenbereich leicht an der Grenzwand haftet. Dadurch wird verhindert, dass der Schutzüberzug unbeabsichtigt aus der Vertiefung des Applikatorteils fällt, beispielsweise durch Schüttelbewegungen oder Umdrehen des Applikatorteils.

In einer Ausführungsvariante weist die dem Innenraum zugewandte Seite des Mantels mehrere Kanäle auf, die sich von der Membran zum Kragen hin erstrecken. Die Kanäle ermöglichen die Abfuhr von Luft, die aus dem Bereich zwischen der Kontaktfläche und dem mit der Kontaktfläche in Kontakt kommenden Kontaktbereich des Schutzüberzugs verdrängt wird. Insbesondere wird die Abfuhr der verdrängten Luft ermöglicht, wenn die Spitze in den Schutzüberzug eingeführt ist und der Mantel auf der Spitze anliegt.

Vorzugsweise ist der Schutzüberzug aus einem Elastomer hergestellt. Der Stempel ist ebenfalls aus einem Elastomer hergestellt und weist einen Andruckbereich mit glatter Oberfläche auf. Der mit der Kontaktfläche in Kontakt kommende Kontaktbereich des Schutzüberzugs ist als Membran mit beidseitig glatter Oberfläche ausgestaltet. Die Verwendung eines Elastomers für den Schutzüberzug liefert einerseits die Dehnbarkeit und Elastizität des Schutzüberzugs und andererseits kann durch Strukturierung der Oberfläche des Schutzüberzugs deren Haftwirkung kontrolliert werden. Eine mit Erosionsstruktur versehene, aufgerauhte Oberfläche weist eine kleine Haftwirkung auf, während eine polierte, glatte, Oberfläche eine grosse Haftwirkung aufweist. Die glatten Oberflächen des Andruckbereichs des Stempels und der Membran bewirken, dass auch kleinste Lufteinschlüsse im Bereich zwischen der Kontaktfläche und dem mit der Kontaktfläche in Kontakt kommenden Kontaktbereich des Schutzüberzugs verdrängt werden. Die glatte Oberfläche der Membran bewirkt zudem, dass der Schutzüberzug mit der Membran optimal an der Kontaktfläche der Spitze haftet.

Die vorliegende Erfindung bezieht sich neben dem Applikationssystem auch auf ein Verfahren zum Anbringen des elastischen Schutzüberzugs auf ein Geräteteil, das eine sich zu einer auf ein Auge aufsetzbaren Kontaktfläche konisch verjüngende Spitze aufweist. Gemäss dem vorgeschlagenen Verfahren wird die Spitze durch eine Öffnung eines Applikatorteils, in den Schutzüberzug eingeführt, der in einer Vertiefung des Applikatorteils oberhalb eines komprimierbaren Stempels des Applikatorteils angeordnet ist. Der beim Einführen mit der Kontaktfläche in Kontakt kommende Kontaktbereich des Schutzüberzugs wird zwischen der Kontaktfläche und dem Stempel gepresst, so dass Luft, die sich zwischen dem Kontaktbereich des Schutzüberzugs und der Kontaktfläche befindet, aus dem Kontaktbereich verdrängt wird. Vorzugsweise wird ein Stempel verwendet, der einen konvexen Andruckbereich aufweist, und beim Einführen wird der mit der Kontaktfläche in Kontakt kommende Kontaktbereich des Schutzüberzugs zwischen der Kontaktfläche und dem Stempel zunehmend gepresst, so dass der mit der Kontaktfläche in Kontakt kommende Kontaktbereich des Schutzüberzugs beim Einführen mit zunehmender Komprimierung des Stempels in einem sich von einem ersten Kontaktpunkt aus vergrössernden Pressbereich zwischen der Kontaktfläche und dem Stempel gepresst wird. Vorzugsweise wird die Spitze in einen sich zum Kontaktbereich des Schutzüberzugs konisch verjüngenden Innenraum des Schutzüberzugs eingeführt bis die Spitze an einen Haftbereich mit glatter Oberfläche angedrückt wird, der sich in einem dem Innenraum zugewandten Bereich eines Kragens des Schutzüberzugs befindet und glatter ist als die Oberfläche des dem Innenraum zugewandten verbleibenden Bereichs des Schutzüberzugs. Dann wird die Spitze mit dem mittels des Haftbereichs an der Spitze haftenden Schutzüberzug durch die Öffnung aus der Vertiefung des Applikatorteils herausgezogen.

Die vorliegende Erfindung bezieht sich schliesslich auch auf ein Applikatorteil wie es oben im Zusammenhang mit dem Applikationssystem beschrieben wurde.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:

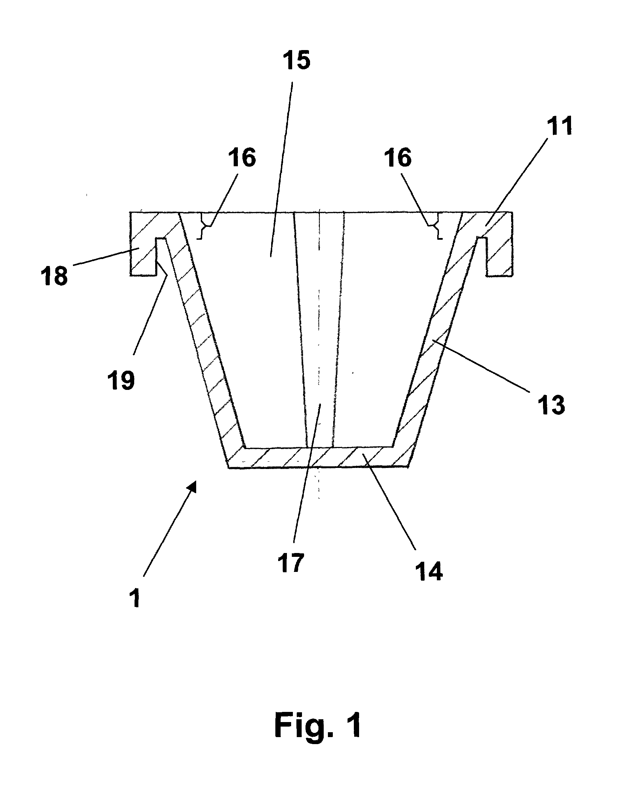

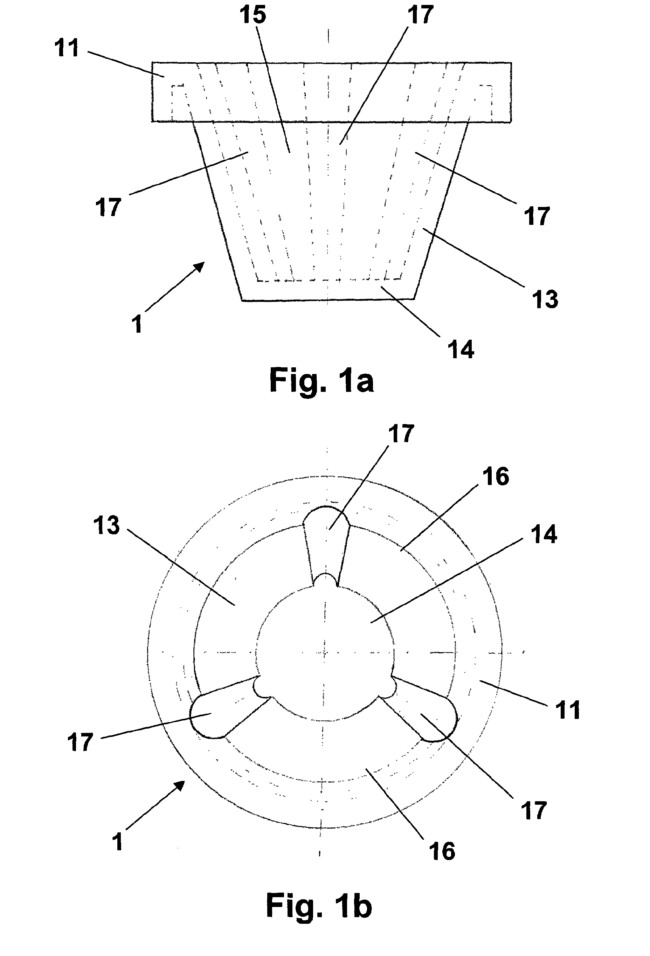

Figur 1 zeigt einen Querschnitt, der schematisch einen elastischen Schutzüberzug entlang der Symmetrieachse illustriert.
Figur 1 a zeigt eine Ansicht, die schematisch den elastischen Schutzüberzug mit angedeuteten Kanälen illustriert.
Figur 1 b zeigt eine Aufsicht, die schematisch den elastischen Schutzüberzug mit den Kanälen illustriert.
Figur 2 zeigt einen Querschnitt, der schematisch ein Applikatorteil illustriert.
Figur 3 zeigt einen Querschnitt, der schematisch das Applikatorteil mit einem darin aufgenommenen Schutzüberzug illustriert.
Figur 4 zeigt einen Querschnitt, der schematisch das Applikatorteil mit einem darin aufgenommenen Schutzüberzug und einer in den Schutzüberzug eingeführten Spitze eines ophthalmologischen Geräteteils illustriert.
Figur 5 zeigt einen Querschnitt, der schematisch die Spitze des ophthalmologischen Geräteteils mit daran angebrachtem Schutzüberzug illustriert.
Figur 6 zeigt einen Querschnitt. Der schematisch eine Spitze mit konkaver Kontaktfläche und daran angebrachtem Schutzüberzug illustriert.
Figur 7a zeigt einen Querschnitt, der schematisch den beim Einführen auftretenden ersten Kontaktpunkt zwischen Membran und Stempel illustriert.
Figur 7b zeigt einen Querschnitt, der schematisch den beim Andrücken sich vergrössernden Pressbereich zwischen Membran und Stempel illustriert.

### Wege zur Ausführung der Erfindung

In den Figuren 1, 1 a, 1b, 2, 3, 4, 5, 6, 7a und 7b werden einander entsprechende, gleiche Komponenten durch gleiche Bezugszeichen bezeichnet.

In den Figuren 1, 1 a, 1b, 2, 3, 4, 5, 6, 7a und 7b bezeichnet das Bezugszeichen 1 einen elastischen Schutzüberzug aus einem Elastomer, beispielsweise Silikon, Polyurethan, weiches PVC oder Kautschuk. Wie aus der Figur 1 ersichtlich ist, ist der Schutzüberzug 1 becherförmig ausgestaltet und weist einen als Membran 14 ausgestalteten kreisrunden Boden, einen mit der Membran 14 verbundenen umlaufenden Mantel 13 sowie einen mit dem Mantel 13 verbundenen umlaufenden Kragen 11 auf. Der Kragen 11 weist im wesentlichen ein U-förmiges Profil auf, das rund oder eckig ausgestaltet ist. Der Kragen 11 bildet einen die Öffnung des Schutzüberzugs 1 umlaufenden Rand, der vom Mantel 13 in der vom Innenraum 15 abgewandten Richtung absteht. Der Durchmesser der Öffnung des Schutzüberzugs 1 beträgt beispielsweise 9.8mm. Die Membran 14, weist vorzugsweise beidseitig eine hochglanzpolierte, glatte Oberfläche auf. Die Membran 14 hat eine konstante Dicke im Bereiche von 40µm bis 50µm, beispielsweise 45µm. Der Durchmesser der Membran 14 beträgt beispielsweise 8mm. Der durch die Membran 14 und den Mantel 13 gebildete Innenraum 15 des Schutzüberzugs 1 verjüngt sich konisch vom Kragen 11 zur Membran 14 und ist bei planer Membran 14 im wesentlichen kegelstumpfförmig. Wie in den Figuren 1 a und 1 b schematisch dargestellt ist, weist der dem Innenraum 15 zugewandte Innenbereich des Mantels 13 mehrere Kanäle 17 auf, die sich jeweils gradlinig von der Membran 14 zum Kragen 11 hin erstrecken. Die Kanäle 17 erstrecken sich gradlinig weiter durch mindestens einen Teil des dem Innenraum 15 zugewandten Innenbereichs des Kragens 11. Obwohl dies in den Figuren 1 a und 1 b nicht dargestellt ist, weist der Mantel 13 beispielsweise acht symmetrisch angeordnete Kanäle 17 auf. Der Mantel 13 hat beispielsweise eine Wandstärke, die sich von 0.5mm im Bereich des Kragens 11 kontinuierlich auf 0.1 mm im an die Membran 14 angrenzenden Bereich verjüngt. Der Übergang vom Mantel 13 zur Membran 14 ist beispielsweise gerundet, zum Beispiel mit einem Innenradius von 0.3mm und einem Aussenradius von 0.35mm. Der Schutzüberzug 1 weist eine Höhe, von der Membran 14 bis zu seiner Öffnung, von beispielsweise 10mm auf.

Der dem Innenraum 15 zugewandte Innenbereich des Kragens 11 weist einen hochglanzpolierten, glatten Haftbereich 16 auf. Der Haftbereich 16 weist beispielsweise umlaufend eine Breite von 0.5 bis 2 Millimetern auf. Das äussere Kragenteil 18 weist einen dem Innenraum 15 zugewandten hochglanzpolierten, glatten äusseren Krageninnenbereich 19 auf.

Ausser an den oben erwähnten Stellen, nämlich den beiden Seiten der Membran 14, dem Haftbereich 16 und dem äusseren Krageninnenbereich 19, weist der Schutzüberzug 1 eine durch Erosionsstrukturen aufgerauhte Oberfläche auf.

Der Schutzüberzug 1 wird mittels eines Heisskanalwerkzeugs einstückig hergestellt. Der Schutzüberzug 1 wird einstückig im Spritzgussverfahren mit anschliessender Umformung hergestellt. Die Oberflächenbeschaffenheit des Schutzüberzugs 1 wird durch entsprechende Oberflächenstrukturen der verwendeten Formteile des Heisskanalwerkzeugs bestimmt. Die entsprechenden Oberflächenbereiche der Formteile zur Bildung der Membran 14, des Haftbereichs 16 und des äusseren Krageninnenbereichs 19 sind hochglanzpoliert. In einem ersten Schritt wird flüssiges elastisches Material in den Hohlraum zwischen den inneren und äusseren Formteilen eingespritzt. Im Moment des Einspritzens befindet sich das innere Formteil im Innern des äusseren Formteils nicht in der Nennposition der Membrandicke sondern beispielsweise etwa 300µm davor. Nach dem Einspritzen des elastischen Materials, wenn der Hohlraum zwischen den Formteilen gefüllt ist, wird das innere Formteil in die Nennposition gebracht, so dass die hochglanzpolierten Oberflächenbereiche zur Bildung einer Membrandicke von 50µm einander auf einen Abstand von 50µm angenähert werden. Das dabei verdrängte elastische Material fliesst ab. Die daraus entstehenden Anschlussstellen werden nach der Aushärtung des elastischen Materials mechanisch entfernt.

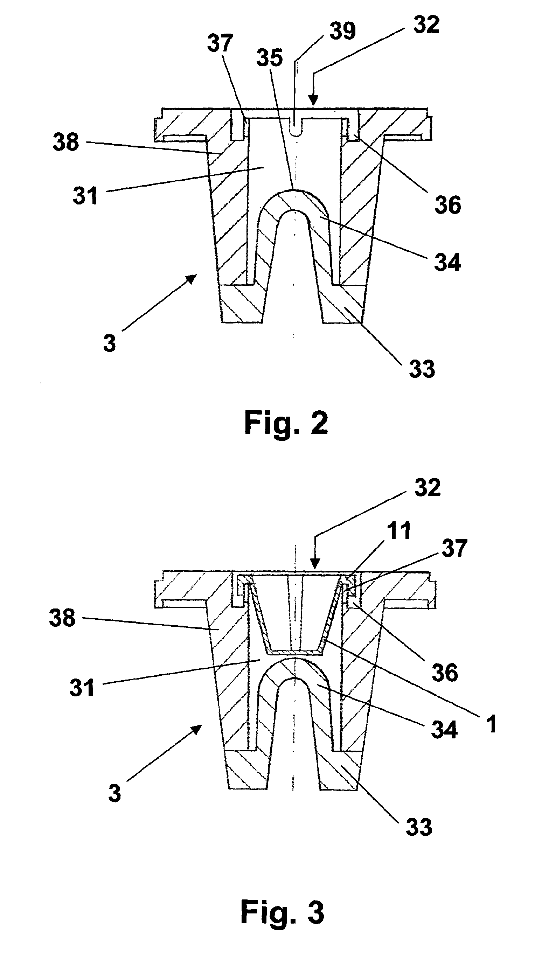

In den Figuren 2, 3 und 4 bezieht sich das Bezugszeichen 3 auf ein Applikatorteil. Das Applikatorteil 3 weist eine Vertiefung 31 mit einer Öffnung 32 und einem Bodenteil 33 auf. Der Durchmesser der Öffnung 32 des Applikatorteils 3 beträgt beispielsweise 10.9mm. Am Bodenteil 33 ist ein Stempel 34 aus komprimierbarem Material angebracht. Das Bodenteil 33 und der Stempel sind beispielsweise einteilig aus einem Elastomer hergestellt. Der Trägerteil 38 des Applikatorteils 3 ist beispielsweise aus einem nicht deformierbaren, harten Kunststoff hergestellt. Das Bodenteil 33 mit dem daran angebrachten Stempel 34 sind am Trägerteil befestigt, beispielsweise mittels eines Zweikomponentenspritzgussverfahrens oder durch Verklebung. Der Stempel 34 hat einen konvexen Andruckbereich 35 und ist beispielsweise gerundet. Damit die Membran 14 vom Stempel 34 möglichst vollständig kontaktiert werden kann, ist der Durchmesser des Stempels 34 vorzugsweise mindestens so gross wie der Durchmesser der Membran 14. Im die Öffnung 32 umlaufenden Randbereich weist das Applikatorteil 3 zudem eine umlaufende Nut 36 auf. Die Nut 36 wird durch eine Grenzwand 37 von der Öffnung 32 getrennt. Die Breite der Nut 36 beträgt beispielsweise 1.8mm. Die Grenzwand 37 kann auch mit Aussparungen 39 versehen sein. Die Wandstärke der Grenzwand 37 beträgt beispielsweise 0.45mm. Der Teil des Randbereichs, der auf der Seite der Nut 36 liegt, die der Grenzwand 37 gegenüberliegt, ist im Bezug zum Bodenteil 33 höher ausgestaltet als die Grenzwand 37, so dass eine entfernbare Abdeckfolie auf dem höheren Teil des Randbereichs 34 angebracht werden kann.

Wie aus der Figur 3 ersichtlich ist, ist das Applikatorteil 3 so beschaffen, dass es den Schutzüberzug 1 in der Vertiefung 31 aufnehmen kann. Das Applikationssystem, dass dem Anwender zur Verfügung gestellt wird, besteht aus einem Applikatorteil 3 und einem darin aufgenommenen sterilen Schutzüberzug 1. Der Schutzüberzug 1 ist in der Vertiefung 31 oberhalb des Stempels 34 angeordnet und wird mittels des Kragens 11 berührungsfrei über dem Stempel 34 gehalten. Das äussere Kragenteil 18 verläuft in der Nut 36 und hält den Schutzüberzug 1 zentrisch in der Vertiefung 31. Der glatte äussere Krageninnenbereich 19 steht in Berührkontakt mit der von der Öffnung 32 abgewandten Seite der Grenzwand 37 und bewirkt so eine trennbare Haftung des Schutzüberzugs 1 am Applikatorteil 3. Durch die oben erwähnte entfernbare Abdeckfolie kann die Öffnung 32 verschlossen und der in der Vertiefung 31 angebrachte sterile Schutzüberzug 1 abgedeckt und vor Verunreinigung geschützt werden.

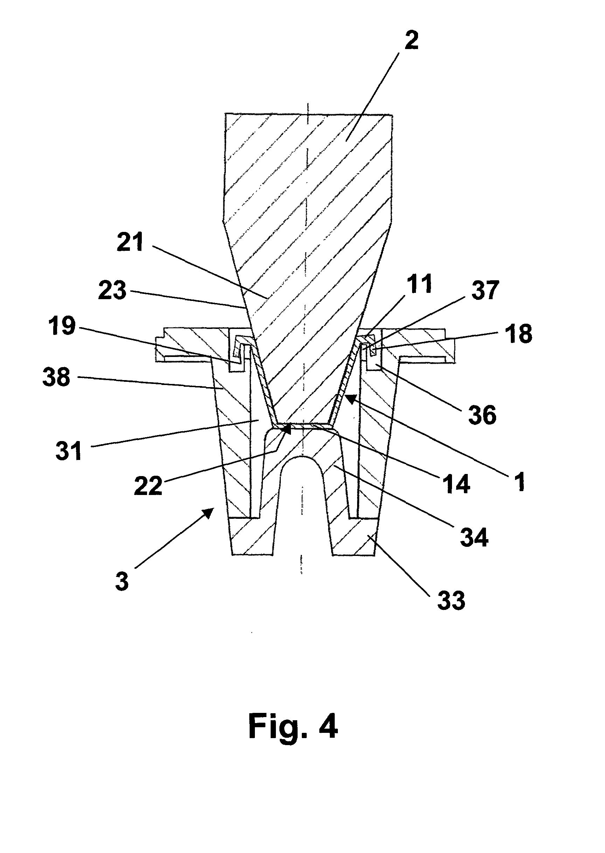

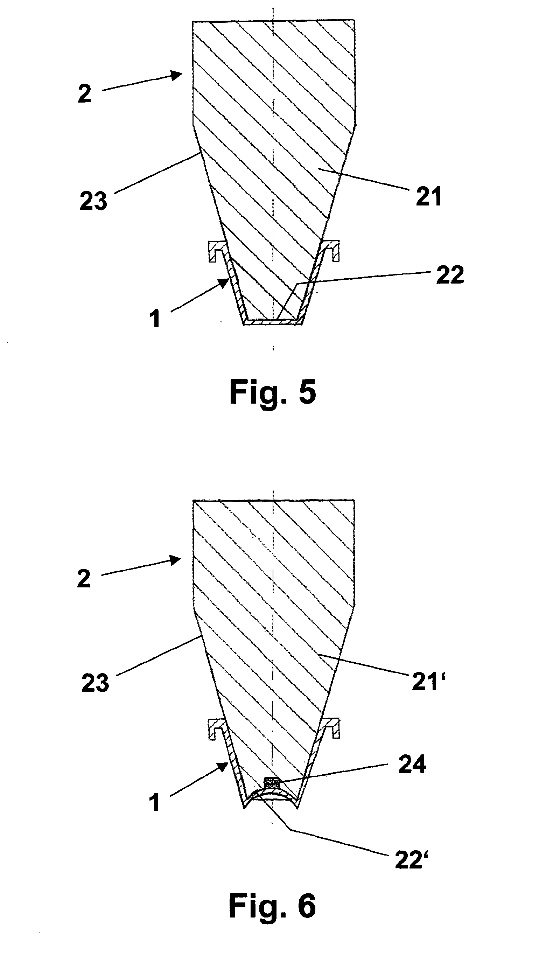

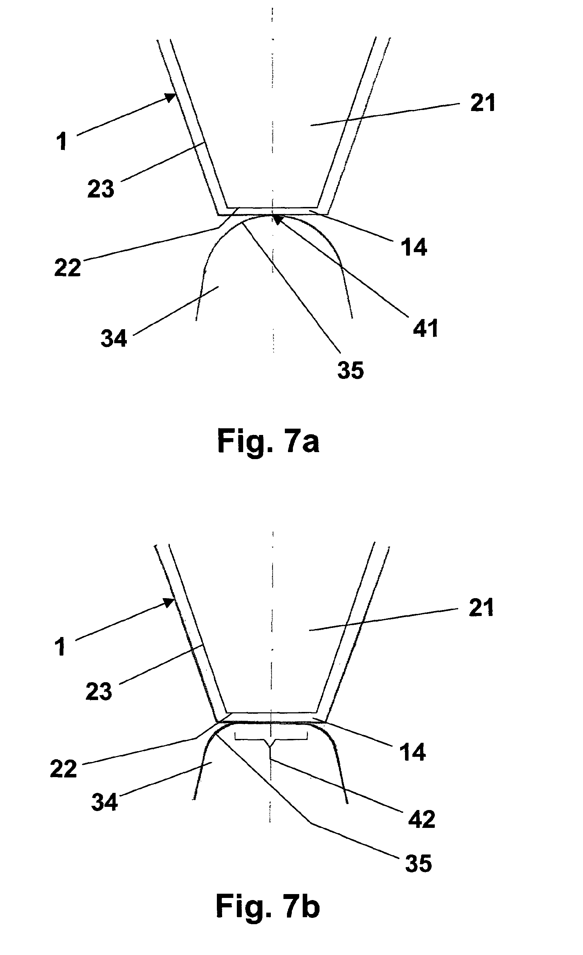

Zum Anbringen des Schutzüberzugs 1 auf die Spitze 21 des ophthalmologischen Geräteteils 2 wird die Abdeckfolie vom Applikatorteil 3 entfernt und die Spitze 21 durch die Öffnung 32 in den in der Vertiefung 31 angebrachten sterilen Schutzüberzug 1 eingeführt. Beim Einführen kommt die dem Innenraum 15 zugewandte Seite der Membran 14 in Kontakt mit der Kontaktfläche 22 der Spitze 21. Durch Weiterführen der Einführbewegung und durch Ausüben von leichtem Druck wird die Membran 14 im Andruckbereich 35 an den Stempel 34 angedrückt. Wie in der Figur 4 ersichtlich ist, wird der Stempel 34 durch weitere Druckausübung mit der Spitze 21 deformiert und die Membran 14 wird zwischen der Kontaktfläche 22 und dem Andruckbereich 35 gepresst. In den Figuren 7a und 7b wird dieser Andruck- und Pressvorgang am Beispiel eines Stempels 34 mit konvexem Andruckbereich 35 illustriert. Beim Einführvorgang berühren sich die Membran 14 und der Andruckbereich 35 des Stempels 34 zunächst im Kontaktpunkt 41 (Figur 7a). Durch weiteres Andrücken der Membran 14 mittels der Spitze 21 vergrössert sich der Pressbereich 42 (Figur 7b) ausgehend vom ersten Kontaktpunkt 41 kontinuierlich. Durch diesen Andruckvorgang mit fortschreitend grösserem Pressbereich 42 wird Luft, die zwischen der Membran 14 und der Kontaktfläche eingeschlossen ist, aus dem Pressbereich 42 verdrängt und über den Aussenbereich 23 der Spitze 21, insbesondere über die oben beschriebenen Kanäle 17, weggeführt. Das Applikatorteil 3 und der Schutzüberzug 1 sind so dimensioniert, dass im Zustand, in dem die Membran 14 zwischen der Kontaktfläche 22 und dem Andruckbereich 35 gepresst wird, der Aussenbereich 23 der Spitze 21 an den Haftbereich 16 des Kragens 11 angedrückt wird und gleichzeitig der glatte äussere Krageninnenbereich 19 von der Grenzwand 37 getrennt wird (Figur 4). Dadurch wird einerseits die Haftung des Kragens 11 von der Grenzwand 37 gelöst und andererseits eine Haftung des Haftbereichs 16 am Aussenbereich 23 der Spitze 21 bewirkt. Der Schutzüberzug 1 haftet sowohl mit dem Haftbereich 16 am Aussenbereich 23 der Spitze 21 als auch mit der Membran 14 an der Kontaktfläche 22 der Spitze 21. Wie in der Figur 5 dargestellt wird, kann die Spitze 21 mit dem daran haftenden Schutzüberzug 1 durch die Öffnung 32 aus der Vertiefung 31 herausgezogen werden. Sowohl das Applikatorteil 3 als auch der Schutzüberzug 1 können nach Gebrauch weggeworfen oder recycliert werden.

An dieser Stelle soll festgehalten werden, dass das beschriebene Applikationssystem und Applikationsverfahren nicht nur für ebene Kontaktflächen 22 geeignet sind, sondern auch für konkave oder konvexe Kontaktflächen anwendbar sind. Der Krümmungsradius des Andruckbereichs 35 des Stempels 34 sollte allerdings kleiner als der Krümmungsradius der Kontaktfläche 22 sein. Figur 6 zeigt ein Beispiel, in welchem der Schutzüberzug 1 auf eine Spitze 21' eines Konturtonometers nach EP 1250884 angebracht ist, das eine konkave Kontaktfläche 22' aufweist, in die ein Drucksensor 24 mit gleicher Kontur wie die konkave Kontaktfläche 22' eingelassen ist.

Abschliessend soll angeführt werden, dass das vorgeschlagene Applikationssystem und Applikationsverfahren selbst bei Kontaktflächen 22 anwendbar sind, die Oberflächen mit Stufen, Absätzen und/oder Spalten aufweisen, wenn diese ohne Beeinträchtigung der Messung von einer stetigen Kurvenform überspannt werden dürfen und in dem gestuften Bereich Luft zwischen dem Schutzüberzug 1 und der Kontaktfläche 22 eingeschlossen sein darf.

Das vorgeschlagene Applikationssystem und Applikationsverfahren sind insbesondere für ophthalmologische Geräteteile wie Messköpfe, beispielsweise Tonometer, insbesondere Konturtonometer mit Drucksensoren, und optischen Applikatoren, insbesondere Pachymeter, geeignet.

## Patentansprüche

1. Applikationssystem, umfassend:
einen elastischen Schutzüberzug (1) für ein ophthalmologisches Geräteteil (2), welches eine sich zu einer auf ein Auge aufsetzbaren Kontaktfläche (22) konisch verjüngende Spitze (21) aufweist, und
ein Applikatorteil (3) zum Anbringen des Schutzüberzugs (1) auf die Spitze (21),
**dadurch gekennzeichnet,**
**dass** das Applikatorteil (3) eine Vertiefung (31) mit einer Öffnung (32) und einem Bodenteil (33) aufweist,
**dass** am Bodenteil (33) ein Stempel (34) aus komprimierendem Material angebracht ist, der vom Bodenteil (33) in Richtung der Öffnung (32) ausgerichtet ist, und
**dass** der Schutzüberzug (1) oberhalb des Stempels (34) in der Vertiefung (31) so entfernbar angeordnet ist, dass die Spitze (21) zum Anbringen des Schutzüberzugs (1) durch die Öffnung (32) in den Schutzüberzug (1) einführbar ist, wobei ein beim Einführvorgang mit der Kontaktfläche (22) in Kontakt kommender Kontaktbereich des Schutzüberzugs (1) zwischen der Kontaktfläche (22) und dem Stempel (34) gepresst wird und Luft, die sich zwischen dem Kontaktbereich des Schutzüberzugs (1) und der Kontaktfläche (22) befindet, aus dem Kontaktbereich verdrängt wird.

2. Applikationssystem nach Anspruch 1, **dadurch gekennzeichnet, dass** der Stempel (34) einen konvexen Andruckbereich (35) aufweist, so dass der beim Einführvorgang der Kontaktfläche (22) in Kontakt kommende Kontaktbereich des Schutzüberzugs (1) mit zunehmender Komprimierung des Stempels (34) in einem sich von einem ersten Kontaktpunkt (41) aus vergrössernden Pressbereich (42) zwischen der Kontaktfläche (22) und dem Stempel (34) gepresst wird.

3. Applikationssystem nach einem der Ansprüche 1 oder 2, **dadurch gekennzeichnet, dass** der Schutzüberzug (1) einen Kragen (11) aufweist, mit dem der Schutzüberzug (1) an einem die Öffnung (32) umlaufenden Randbereich des Applikatorteils (3) über dem Stempel (34) gehalten wird, dass der Schutzüberzug (1) einen Mantel (13) aufweist, welcher einen sich vom Kragen (11) zum Kontaktbereich des Schutzüberzugs (1) konisch verjüngenden Innenraum (15) bildet.

4. Applikationssystem nach Anspruch 3, **dadurch gekennzeichnet, dass** der Kontaktbereich des Schutzüberzugs (1) als Membran (14) ausgestaltet ist, und dass der Kragen (11) einen dem Innenraum (15) zugewandten Haftbereich (16) mit glatter Oberfläche aufweist, die glatter ist als die Oberfläche der dem Innenraum (15) zugewandten Seite des Mantels (13).

5. Applikationssystem nach einem der Ansprüche 3 oder 4, **dadurch gekennzeichnet, dass** der Randbereich eine die Öffnung (32) umlaufende Nut (36) aufweist, dass die Nut (36) durch eine Grenzwand (37) des Applikatorteils (3) von der Öffnung (32) abgegrenzt ist, dass der Kragen (11) ein in der Nut (36) verlaufendes äusseres Kragenteil (18) umfasst, und dass das äussere Kragenteil (18) einen der Öffnung (32) zugewandten äusseren Krageninnenbereich (19) mit glatter Oberfläche aufweist, der die Grenzwand (37) kontaktiert.

6. Applikationssystem nach einem der Ansprüche 3 bis 5, **dadurch gekennzeichnet, dass** die dem Innenraum (15) zugewandte Seite des Mantels (13) mehrere Kanäle (17) aufweist, die sich vom Kontaktbereich zum Kragen (11) hin erstrecken.

7. Applikationssystem nach einem der Ansprüche 1 bis 6, **dadurch gekennzeichnet, dass** der Schutzüberzug (1) aus einem Elastomer hergestellt ist, dass der Stempel (34) aus einem Elastomer hergestellt ist, dass der Stempel (34) einen Andruckbereich (35) mit glatter Oberfläche aufweist, und dass der mit der Kontaktfläche (22) in Kontakt kommende Kontaktbereich des Schutzüberzugs (1) als Membran (14) mit beidseitig glatter Oberfläche ausgestaltet ist.

8. Verfahren zum Anbringen eines elastischen Schutzüberzugs (1) auf ein ophthalmologisches Geräteteil (2), das eine sich zu einer auf ein Auge aufsetzbaren Kontaktfläche (22) konisch verjüngende Spitze (21) aufweist, **gekennzeichnet durch**,
Einführen der Spitze (21) **durch** eine Öffnung (32) eines Applikatorteils (3), in den Schutzüberzug (1), der in einer Vertiefung (31) des Applikatorteils (3) oberhalb eines komprimierbaren Stempels (34) des Applikatorteils (3) angeordnet ist,
Pressen eines beim Einführen mit der Kontaktfläche (22) in Kontakt kommenden Kontaktbereichs des Schutzüberzugs (1) zwischen der Kontaktfläche (22) und dem Stempel (34), so dass Luft, die sich zwischen dem Kontaktbereich des Schutzüberzugs (1) und der Kontaktfläche (22) befindet, aus dem Kontaktbereich verdrängt wird.

9. Verfahren nach Anspruch 8, **gekennzeichnet durch**, Verwenden eines Stempels (34), der einen konvexen Andruckbereich (35) aufweist, und zunehmendes Pressen des beim Einführen mit der Kontaktfläche (22) in Kontakt kommenden Kontaktbereichs des Schutzüberzugs (1) zwischen der Kontaktfläche (22) und dem Stempel (34), so dass der mit der Kontaktfläche (22) in Kontakt kommende Kontaktbereich des Schutzüberzugs (1) mit zunehmender Komprimierung des Stempels (34) in einem sich von einem ersten Kontaktpunkt (41) aus vergrössernden Pressbereich (42) zwischen der Kontaktfläche (22) und dem Stempel (34) gepresst wird.

10. Verfahren nach einem der Ansprüche 8 oder 9, **gekennzeichnet durch**, Einführen der Spitze (21) in einen sich zur Kontaktfläche (22) konisch verjüngenden Innenraum (15) des Schutzüberzugs (1) bis die Spitze (21) an einen Haftbereich (16) mit glatter Oberfläche angedrückt wird, welcher Haftbereich (16) sich in einem dem Innenraum (15) zugewandten Bereich eines Kragens (11) des Schutzüberzugs (1) befindet und glatter ist als die Oberfläche des dem Innenraum (15) zugewandten verbleibenden Bereichs des Schutzüberzugs (1), wobei der Schutzüberzug (1) **durch** den Kragen (11) an einem die Öffnung (32) umlaufenden Randbereich des Applikatorteils (3) über dem komprimierbaren Stempel (34) gehalten wird, und Herausziehen der Spitze (21) mit dem mittels des Haftbereichs (16) an der Spitze (21) haftenden Schutzüberzug (1) **durch** die Öffnung (32) aus der Vertiefung (31) des Applikatorteils (3).

11. Applikatorteil (3) zum Anbringen eines elastischen Schutzüberzugs (1) auf eine sich zu einer auf ein Auge aufsetzbaren Kontaktfläche (22) konisch verjüngende Spitze (21) eines ophthalmologischen Geräteteils (2),
**dadurch gekennzeichnet,**
**dass** das Applikatorteil (3) eine Vertiefung (31) mit einer Öffnung (32) und einem Bodenteil (33) zum Aufnehmen des Schutzüberzugs (1) aufweist,
**dass** am Bodenteil (33) ein Stempel (34) aus komprimierendem Material angebracht ist, der vom Bodenteil (33) in Richtung der Öffnung (32) ausgerichtet ist, und
**dass** zum Anbringen des Schutzüberzugs (1) die Spitze (21) durch die Öffnung (32) so in einen in der Vertiefung (31) angebrachten Schutzüberzug (1) einführbar ist, dass ein beim Einführvorgang mit der Kontaktfläche (22) in Kontakt kommender Kontaktbereich des in der Vertiefung (31) angebrachten Schutzüberzugs (1) zwischen der Kontaktfläche (22) und dem Stempel (34) gepresst wird und Luft, die sich zwischen dem Kontaktbereich des Schutzüberzugs (1) und der Kontaktfläche (22) befindet, aus dem Kontaktbereich verdrängt wird.

12. Applikatorteil (3) nach Anspruch 11, **dadurch gekennzeichnet, dass** der Stempel (34) einen konvexen Andruckbereich (35) aufweist, so dass der beim Einführvorgang der Kontaktfläche (22) in Kontakt kommende Kontaktbereich des in der Vertiefung (31) angebrachten Schutzüberzugs (1) mit zunehmender Komprimierung des Stempels (34) in einem sich von einem ersten Kontaktpunkt (41) aus vergrössernden Pressbereich (42) zwischen der Kontaktfläche (22) und dem Stempel (34) gepresst wird.
